# EUROPEAN PATENT SPECIFICATION

(11) **EP 0 348 061 B1**
(45) Date of publication and mention of the grant of the patent: **25.10.1995**
(21) Application number: 89305662.2
(22) Date of filing: 05.06.1989
(51) Int. Cl.: A61F 2/00, A61C 13/00, B29C 35/08

(54) **Stereolithographic Process for Preparing Prosthetic Devices**
Stereolithographisches Verfahren zur Herstellung von Prothesen
Procédé stéréolithographique de fabrication de prothèses

(30) Priority: 24.06.1988 GB 8815065
(43) Date of publication of application: 27.12.1989
(73) Proprietor: ZENECA LIMITED, London W1Y 6LN (GB)
(72) Inventor: Jones, Michael Edward Benet, Viscars Cross Chester (GB); Thomas, Ieuan, Frodsham Cheshire WA6 6QD (GB)
(74) Representative: Stephenson, Kenneth

(56) References cited:
- EP-A- 0 054 785
- EP-A- 0 090 493
- EP-A- 0 146 191
- EP-A- 0 162 651
- US-A- 4 575 330

## Description

This invention relates to a stereolithographic process for preparing prosthetic devices.

In our EP-A-0 339 816 we describe prosthetic devices which comprise a composite prepared by curing certain addition-polymerisable compositions defined therein. The aforesaid curing is effected by known techniques, in a mould of appropriate shape and size. It will be appreciated that variation between individuals can lead to the needs inter alia (A) to prepare a mould to suit a particular individual in whose body the device is to be disposed or (B) to adapt a pre-made product to the requirements of a particular individual in whose body the device is to be disposed.

In the aforesaid need (A), a dental crown, for example, is made specifically for a particular tooth residue in a particular individual.

Typically, dental crowns are prepared from a ceramic, or a reinforced polymer or a metal, e.g. gold. In the conventional procedure for the preparation of dental crowns: (a) a soft rubber is cast around the residue of the tooth which is to carry the crown to produce a mould of the residue; (b) Plaster of Paris, for example, is cast into the mould to prepare a hard model of the tooth residue; and (c) a crown of appropriate material is "built", using the Plaster of Paris model as a template. This tends to be a painstaking process involving considerable time, effort and expense. The finished crown is reviewed and the laborious process often has to be repeated to produce a crown of the desired shape and size.

In the aforesaid need (B), there tends to be a specific number of manufactured replacements, eg hip-joint replacements, which have to be selected to match as closely as possible, a patient's needs. Typically, such matchings are effected by sculpting the patient' s anatomy to accommodate the pre-made device. Alternatively, a trial prosthesis can be contoured to match the recipient zone as described in US 4 506 396.

Moreover, there is a tendency for conventional implants to fail because the cement, eg acrylic, which binds the implant to the bone adjacent thereto breaks down, for example in patients who place greater stress on their joints, eg over-weight or very active patients. Cementless implants have been developed in an attempt to overcome this problem of cement failure. Typically such cementless implants have a porous metal coating into the pores of which bone can grow. However, the correct fit of the implant in the recipient zone is critical.

To obtain the correct fit for example for a hip replacement, it has been suggested that a plurality, typically three, locating devices, so-called "calibration pins", are disposed in the thigh bone which is then scanned by computer-aided tomography. The images so-obtained are combined to form a 3-dimensional model from which the optimal shape, size and location of the implant and its cavity in relation to the calibration pins can be determined. From this data, a robot drills an accurately sized cavity in correct location in the thigh bone.

Recently implants and anatomic models have been manufactured using computed tomography (CT) image data and a system to generate instructions for certain numerically controlled grinding machines (Rhodes et al, IEEE CG&A, February 1987, p13).

In EP-A-0054785, there is described a method for the fabrication of implants, for example a dental inlay, which involves the steps of noncontact topographic mapping of the natural contours of a body organ or of the surfaces, three-dimensional shapes and/or cavities artificially rendered into such organ and of the adjacent, untouched tissue contours by either directly scanning the contours or by scanning a replica of the contours; recording such contours utilizing a computing means and three-dimensional image processing techniques; designing and displaying a data set of the implant required to complement the organ under restoration; completing the data set of the implant by approximating the nonreadable parts necessary to describe a continuous, organ-restoring surface for the implant utilizing empirically determined curve sections fitted to the registered data; and fabricating an alloplastic implant from a material based upon the data set and a machining program, so that the implant optimally conforms to the interior and exterior surfaces of the body organ. The actual fabrication of the implant is effected by cutting, milling or eroding a blank of suitable material using, for example, a numerically controlled milling machine.

We have now devised a process for the preparation of prosthetic devices from curable compositions which process uses computer generated graphics to generate an image of the zone which is to receive the prosthetic device, which zone is hereinafter referred to for convenience as "recipient zone". The process substantially reduces the amount of time and effort involved in the preparation of such devices and gives a good match of the prosthetic device to the recipient zone in, or on, which it is to be disposed. Such custom prostheses are particularly useful for patients who require revision, have congenital degenerative hip disease, need limb salvage due to neoplastic or traumatic disorder or are young and very active.

In computer generated graphics displayed on a cathode-ray tube (CRT), the image is a picture of a computer coded object. The skilled man can work with the image to design a desired product. When he is satisfied with the product as displayed on the screen, the computer can be used to generate the programme commands and transmit the programme signals suitable for production of the product.

According to a first aspect of the present invention there is provided a process for the preparation of a prosthetic device which process comprises the steps of:
- Step A:: generating an image of the recipient zone which is to receive the prosthetic device;
- Step B:: designing the prosthetic device from the image;
- Step C:: translating the shape and size of the prosthetic device designed in Step B into commands that are appropriate for controlling a source of electromagnetic radiation;
- Step D:: delivering the commands from Step C to, and to control, the source of electromagnetic radiation such that a beam therefrom defines the shape and size of the prosthetic device; and
- Step E:: exposing a fluid addition-polymerisable composition to the beam from Step D such that a solid of the shape and size of the prosthetic device is prepared therefrom;
wherein in Step E of the process the addition-polymerisable composition is exposed to suitable electromagnetic radiation such that a solid lamella representing a cross-section of the device is produced; and
wherein successive adjacent lamellae as they are formed are cohesively integrated such that they define the shape and size of the prosthetic device; and
wherein the prosthetic device is a joint, a complex fracture repair, a bone plate, a dental crown or a bone reconstructure.

The prosthetic device prepared by the process according to the present invention may be substantially two-dimensional, for example a bone plate, e.g. as an insert in a long bone or a cranial plate, or preferably substantially three-dimensional.

As typical examples of three-dimensional prosthetic devices may be mentioned inter alia joints, e.g. femural head replacement; complex fracture repairs; and bone restructuring in reconstructive surgery, e.g. jaw-bone.

Whereas the process of the present invention is described hereinafter with particular reference to hip-joints, it will be appreciated that replacements for alternative parts of the human anatomy may be prepared hereby. For example, the process of the present invention may be adapted for the preparation of inter alia, shoulders, elbows, knuckles and more complicated arrangements, eg a knee which is believed to combine a hinge action with a gliding and rolling action.

It will be appreciated that the aforesaid recipient zone which is to be imaged will be chosen in the light of inter alia the location and the function of the prosthetic device within the human body. For example, where a dental crown is prepared by the process according to the present invention, the surface of the residual tooth and the disposition and orientation of that tooth residue in relation to adjacent and opposed teeth will define at least a portion of the size, shape, and contour of the prosthetic device; using step C of the process of the present invention the skilled man may then design the device such that it is acceptable, e.g. mechanically and cosmetically, in the recipient location.

Where femural head hip-joint replacement is prepared by the process of the present invention the surface of the lumen of the residual femur will define the size, shape, and contours of a portion of the device, i.e. the shaft. Using Step C of the process of the present invention, the skilled man may then design the device such that the resultant form enhances the acceptability and hence tends to increase the useful life of the device.

Methods for imaging a recipient zone within the human body are known in the art. For example, where a tooth residue is in a condition which is judged to be suitable for receiving a crown, a photograph is taken of at least that part of the surface of the tooth residue which is to receive the crown. US 4,661,967 describes a dental radiographic device for photographing a jaw. It is often preferred that such a photograph is taken by laser light, e.g. as is more fully described by Rekow et al, Ninth Annual Conference of the Engineering in Medicine and Biology Society, p0733,. Although we do not exclude the possibility that the contours of the aforesaid receptor surface can be determined by alternative methods. Details of the aforesaid contours may be fed to and stored in a suitable data base, e.g. a computer.

A variety of systems for the automatic production of dental prostheses are described in Rekow, Ninth Annual Conference of the Engineers, p0733. Each system uses CAD techniques for designing dental restoratives, which techniques are useful in the present invention, but then convert such data relating to the designed dental restorative into CAM commands for the milling of a solid, eg a machineable ceramic.

Where the prosthetic device is an artificial replacement for parts of the anatomy internally resident in the human or animal body, i.e. it is a so-called endoprosthesis, e.g. a femural head replacement, it will be appreciated that x-ray photography is typically used in Step A of the process according to the present invention. Typical x-ray photographic or radiographic equipment and procedures for using it to take photographs of the human body are well known. However, we do not exclude the possibility that imaging technology, so-called "machine-vision", as applied to medical imaging devices such as body scanning systems may be applied. For example, computed tomography (CT) scanners may be used to convert X-rays into digital computer code to make high-resolution video images which depict hard tissue, e.g. bone and teeth, in fine detail. Alternatively, positron emission tomography (PET) or single photon emission computed tomography (SPECT) may be used to produce 3-D images of the recipient zone.

Procedures for converting such photographic data into computer-data are known.

Procedure for using such computer-data in the design of certain engineering products and the manufacture thereof are known, e.g. as described in GB 1,243,043 and US 4,575,330.

Quantitative computed tomography methods have been used in the art to reconstruct the cross-sectional anatomy of individual femurs. Automatic contour detection algorithms and interactive editing have been used to transform this data into 3-D model of the medullary canal (Nelson et al, Bioengineering, Proceedings of the Northeast Conference 1985, published by IEEE, NY, USA, p200-201).

The polymerisable composition used in the process according to the present invention is fluid, that is paste-like or liquid (not powdery and crumblient) such that it flows under the temperature and pressure at which the process is operated. Whereas we do not exclude the possibility that the viscosity of the polymerisable composition is such that it may need to be pumped into the apparatus used in the process, this is not preferred.

It will be appreciated that the polymerisable composition used in the process according to the present invention should have certain properties. For example, (A) it should cure fast enough on exposure to suitable electromagnetic radiation to afford a prosthetic device in a commercially useful preparation time; (B) it should be reasonably soluble in a solvent and the cured product should be reasonably insoluble in the same solvent such that the product can be washed free of the residue of uncured polymerisable composition; and (C) it should be as non-toxic and non-irritating as possible both in its uncured and cured state. Furthermore, where it is used in the process according to the preferred aspect of the present invention: (D) it must be fluid and the viscosity thereof should be such that it can flow across the cured surface of the preceding lamella; (E) it must be adhesive such that successive lamellae adhere to each other; and (F) it should absorb the incident electromagnetic radiation such that thin layers of solid lamellae are formed.

The aforesaid addition-polymerisable composition comprises a component (hereinafter referred to for convenience as "Component A") having at least two addition- polymerisable olefinically unsaturated carbon-carbon double bonds. Component A, which may be neat or an admixture, will be chosen in the light of inter alia (i) the desired processability properties of the polymerisable composition, and (ii) the desired properties, e.g. flexural modulus and cosmetic appearance, of the prosthetic device prepared therefrom.

It is often preferred that the addition-polymerisable composition further comprises at least one particulate inorganic solid. However, we do not exclude the possibility that certain prosthetic devices prepared by the process of the present invention do not comprise the at least one particulate inorganic solid.

As examples of Component A may be mentioned inter alia monomers, e.g. triethylene glycol dimethacrylate, the dimethacrylate of oxyethylated bisphenol A, i.e. 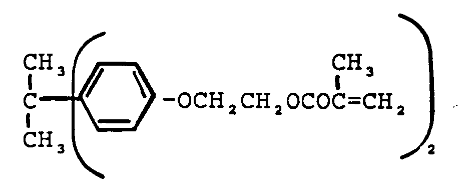
or preferably oligomers. As examples of oligomers may be mentioned inter alia vinyl urethanes, e.g. as described in our GB Patent Specifications Nos 1,352,063 1,465,097 or 1,498,421; unsaturated aromatic compound/aldehyde oligomers, e.g. as described in our European Patent Specification Nos 0,112,650A and 0,162,651A.

The presence of a monomer, in addition to the aforesaid preferred oligomers, in Component A is often preferred, since it tends to reduce the viscosity of the polymerisable composition and hence facilitates the flow thereof in the process according to the preferred aspect of the present invention.

The particulate inorganic solid, where present, should be stiff enough to impart desired mechanical properties to the device. For example, where the device is a dental crown, it should impart abrasion resistance and rigidity to the crown and should be non-staining and non-leachable, i.e. stable for extended periods, e.g. more than 10 years, in a moist environment.

It will be appreciated that the particulate inorganic solid, where present, should not unduly inhibit polymerisation of the polymerisable composition in Step E, e.g. the polymerisable compositions containing the particulate inorganic solid should be sufficiently transparent to the appropriate electromagnetic radiation as hereinafter described. For example, the refractive indices of the particulate inorganic solid and Component A are preferably matched.

As examples of suitable particulate inorganic solids which may be used in the polymerisable composition may be mentioned inter alia mica, talc, alumina, hydroxyapatite or preferably a glass, e.g. a borosilicate, more preferably a radio-opaque glass, e.g. Raysorb T3000 (RTM). It is often preferred that at least a portion of the solid is radio-opaque.

We do not exclude the possibility that a portion, up to 10% say, of the particulate inorganic solid may be in the form of, for example, plates or fibrils.

Preferably, the particulate inorganic solid is treated with a suitable coupling agent to improve the bonding thereof to the cured addition-polymerisable composition. For example, where the particulate inorganic solid is silica or glass it may be treated with a suitable silane coupling agent, e.g. γ-methacrylyl- oxypropyl-trimethoxysilane.

Where the addition-polymerisable composition comprises a dispersion of a particulate inorganic solid therein it is often preferred that the composition comprises a dispersion stabiliser which stabilises the dispersion of the particulate inorganic solid in the composition.

The polymerisable composition preferably comprises 10-80%, more preferably 40-75% and particularly more preferably 65-70% by volume particulate inorganic solid. Above about 80% v/v, it is found that the particulate inorganic solid often cannot be distributed homogeneously in the polymerisable composition and/or that the polymerisable composition is too viscous to flow readily in Step E of the process. Below about 10% v/v, devices prepared by polymerisation of the composition tend to be too compliant in certain applications.

For cosmetic appearance, for example where the prosthetic device is a dental crown, it is often preferred that a particulate ceramic material is present therein, particularly at, or adjacent, the exposed surface thereof.

The polymerisable composition used in the process according to the present invention comprises a catalyst which absorbs the incident electromagnetic radiation and initiates the curing of the polymerisable composition (which catalyst is hereinafter referred to for convenience as "light-curing catalyst"). Preferably a light-curing catalyst as described in our GB Patent Specifications Nos 1,408,265 or 1,494,903 or in our European Patent Specifications Nos 0,059,649 or 0,090,493 is used.

It is often preferred that the light-curing catalyst provides an efficient source of free radicals when irradiated with visible light, e.g. of wavelength greater than 400nm. As suitable sources of electomagnetic radiation may be mentioned inter alia lasers, carbon arcs, mercury vapour arcs and quartz iodide lamps.

It will be appreciated that it will be necessary to use a source which emits electromagnetic radiation of a wavelength to which the light-curing catalyst is sensitive. For example, where one of our aforementioned light-curing catalysts is used, with sensitivity in the blue region, it is often preferred that an Argon Ion laser is used.

We do not exclude the possibility that a plurality, e.g. two, beams of electromagnetic radiation may be directed into a polymerisable composition, which is transmissive to the discrete beams, to intersect at specified coordinates, wherein the discrete beams may be of the same or differing wavelength. The beams may be used sequentially to intersect the same points or simultaneously, only the beam intersection points are stimulated to sufficient energy levels to initiate the curing process for forming a device within the volume of a fluid medium. Where a plurality of beams is employed it is often preferred that the light-curing catalyst comprises two components, a first of which is activable by electromagnetic radiation of a first wavelength and the second of which is activatable by electromagnetic radiation of a second wavelength. Two beams of appropriate different electromagnetic radiation may be directed into the polymerisable composition. Only at the point or zone within the polymerisable compositon where the two beams coalesce or converge does initiation of polymerisation occur. Such two component systems and devices for the use thereof, are more fully described in US 4,041,476.

For example, where the photoinitiator system comprises an amine and a ketone the amine may be used in the form of a protected amine. Radiation of a first wavelength liberates the free amine and radiation of a second wavelength excites the ketone to an excited state in which it reacts with the free amine. Only where the two beams coalesce is polymerisation initiated.

The preferred aspect of the present invention, as is more fully hereinafter described, uses the principles of computer generated graphics in combination with stereolithography, i.e. the application of lithographic techniques, to the production of three-dimensional objects, eg dental crowns, directly from computer instructions.

"Stereolithography" is a method and apparatus for making solid objects by successively "printing" thin layers of a curable material, e.g. a photo-curable material, one on top of the other. A programmed movable spot beam of electromagnetic radiation shining on a surface or layer of curable fluid is used to form a solid cross-section of the object at the surface of the fluid. The solid cross-section is then in a programmed manner moved above, ie withdrawn from, or below, ie lowered into, the working surface by the thickness of one lamella. For example, the solid cross-section is lowered beneath the fluid surface by the thickness of one lamella, and the next solid cross-section is then formed and adhered to the immediately preceding lamella defining the object. Alternatively, the fluid may be present as a thin layer supported on a transparent support, typically a liquid, through which the beam passes to impinge on the fluid. The solid cross-section is then raised above the liquid by the thickness of one lamella. This lamellar preparation process is continued until the device is formed.

The use of computer-aided design and computer aided manufacture (CAD-CAM) in the production of certain three-dimensional engineering products is known and the use of an addition-polymerisable composition therein has been described, e.g. in US 4,288,861, and US 4,575,330.

The devices prepared by the process of the present invention may be employed in the living body in a wide variety of applications and the physical characteristics of the device, its dimensions, chemical nature, etc will be selected in the light of the proposed use.

The devices or a portion thereof, prepared by the process of the present invention, may be provided with an appropriate surface to improve further its intended use, eg increase its useful life. For example, it may be provided with (a) a bearing surface, e.g. of titanium, to improve it wear properties or (b) a tissue-compatible surface to encourage in-growth of living cells.

The process of the present invention is particularly applicable to the preparation of a prosthetic device comprising a plurality of inter-acting parts e.g. total hip-joint replacement, i.e. both ball and socket.

Where a dental crown is produced by the process according to the present invention it may be attached to the tooth residue by conventional methods, for example by a dental cement, e.g. a polycarboxylic resin, and/or by mechanical interaction. It will be appreciated that the accuracy and fidelity of the data generated in Step A in the process according to the present invention may lead to a crown having contours which tend to increase the strength of mechanical bonding between the tooth and the crown.

The process of the present invention may be carried out in situ on location e.g. in a hospital or dental surgery. Typically, however, at least certain steps in the process may be carried out in a central manufacturing laboratory to which the details of the contours and dimensions of the recipient zone or device can be readily transmitted, e.g. on electromagnetic tape or by telecommunication. For example, the initial step, Step A, may be carried out at a first site, further steps, Steps B and C, may be carried out at a second site, and then steps D and E may be carried out at the first site; alternatively Steps B, C, D and E may be carried out at the second site and the product mailed to the first site.

For example, X-ray radiographic data, particularly of hips, knees and fractures, can be generated on location at a discrete hospital. Such data can be transferred, e.g. telecommunicated, to a central manufacturing site where it can be processed to provide input data in a form suitable for carrying out the further steps of the process of the present invention. Alternatively, such date can be generated at a first site and transmitted to a second site where the device is designed. The data defining the designed device can the be transmitted to the first site for manufacture of the device.

The present invention is further illustrated by reference to the followings drawings which show, by way of example only, two embodiments of the process:
In Figure 1, a fluid polymerisable composition is continuously fed to a container; and
in Figure 2, the prosthetic device prepared by the proces is continuously withdrawn from a pool of polymerisable composition.

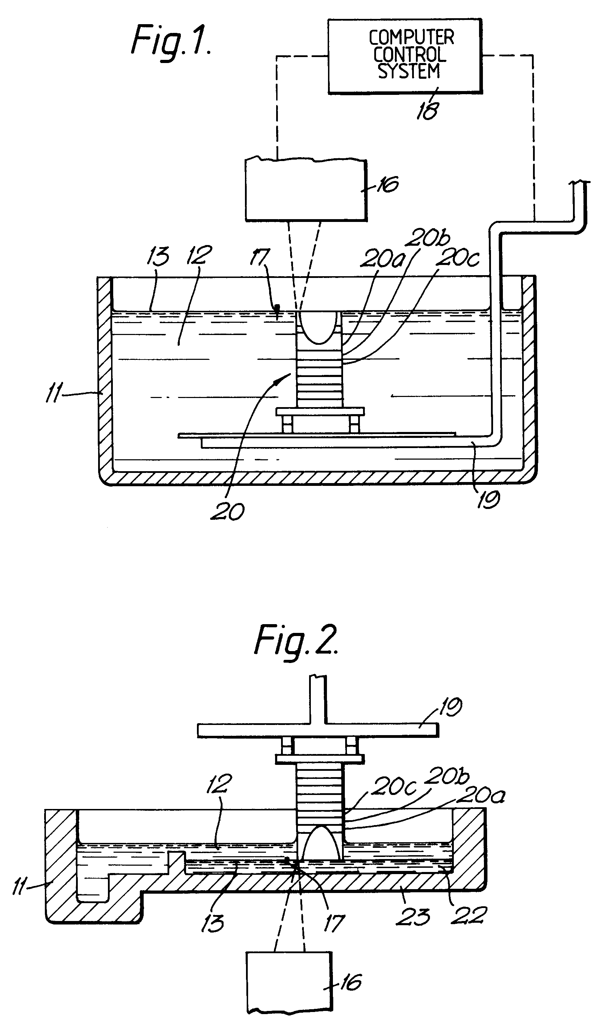

In Figure 1, a container 11 is filled with a pond 12 of a photo-polymerisable composition, and a working surface 13 is formed. A programmable source of electromagnetic radiation 16 (e.g. from a suitable laser) produces a spot of light 17 on the surface 13. The spot 17 is movable across the surface 13 by means known in the art, e.g. motion of mirrors. The position of spot 17 on the surface 13 is controlled by a computer 18. A moveable platform 19 in container 11 can be moved up or down selectively, the position thereof being controlled by computer 18. As the system operates it produces a three-dimensional dental crown 20 by step-wise addition of integrated lamellae 20a, 20b, 20C as the platform 19 descends into the pond 12.

The surface 13 of the pond 12 of the polymerisable composition is maintained at a constant level in the container 11 and the spot of light 17 of sufficient intensity to cure the fluid and convert it into a solid material is moved across the working surface 13 in a programmed manner. As the fluid 12 cures and solid material forms, the elevator platform 19 that was initially just below the surface 13 is moved down from the surface in a programmed manner by a suitable actuator. In this way, the solid material that was initially formed is taken below surface 13 and further fluid 12 flows across the surface 13. A portion of this further fluid is,in turn, converted to solid material by the programmed light spot 17, and the new material adhesively connects to the material below it. This process is continued until the three-dimensional dental crown 20 is formed. The elevator platform 19 is raised and the dental crown removed therefrom. The dental crown is then rinsed, optionally ultrasonically, in a solvent, e.g. acetone, to dissolve residual uncured polymerisable composition.

Referring now to Figure 2, wherein the same numerals indicate the same components identified in Figure 1.

A transparent window 23 is provided in the base of the container 11. The polymerisable composition 12 is provided as a thin layer on a heavier liquid 22. Liquid 22 is non-miscible with, and non-wettable by, the polymerisable composition 12 and is transparent to the electromagnetic radiation from the source 16.

It will be appreciated that where simply shaped, e.g. two-dimensional, devices are prepared the computer may not be necessary and a simpler dedicated programming system can be used. Alternatively, the computer control system can be merely executing instructions that were formed by a further, more complex computer, e.g. where a plurality of stereolithographic units are used to produce the device and another computer is used to design the device.

We do not exclude the possibility that a plurality of containers may be used, each container having a different polymerisable composition that may be automatically selected by the stereolithographic system. For example, sheath-core structures may be prepared in which the sheath provides a coating of a suitable material, e.g. wear-resistant or susceptible to incursion of living cells as described hereinbefore, on a core prepared by the process of the present invention.

The source of electromagnetic radiation produces the spot thereof small enough to allow the desired object detail to be formed, and intense enough to cure the polymerisable composition quickly enough to be commercially useful. The source is arranged such that it can be programmed to be turned off and on and to move, such that the focussed spot moves across the surface of the fluid. Thus, as the spot moves, it cures the fluid into a solid, and "draws" a solid pattern on the surface in much the same way a chart recorder or plotter uses a pen to draw a pattern on paper.

In the system of Figure 1, means may be provided to keep the surface 13 at a constant level and to replenish the polymerisable composition after a prosthetic device has been removed, so that the focus spot 17 will remain sharply in focus on a fixed focus plane, thus improving resolution when forming a thin layer along the working surface. It will be appreciated that it is desired to adapt the focal point such that it provides a region of high intensity at the working surface 13 and rapidly diverges to low intensity and thereby controls the depth of the curing process to provide the thinnest appropriate cross-sectional lamella for the prosthetic device being prepared. Preferably this is accomplished by using a short focal-length lens and bringing the radiation source 16 as close as practically possible to the working surface 12. Maximum divergence then occurs in the cone of focus entering the fluid medium and resolution is substantially enhanced.

A suitable digital plotter may be used to control the radiation source. The lens tube is attached to the pen carriage of the plotter, and the plotter is driven by a computer using conventional graphic commands. The shutter is controlled by computer commands.

Alternative physical forms of the source of electromagnetic radiation or an equivalent thereof may be used. For example, scanning could be done with optical scanners or preferably an appropriate laser light source.

The skilled man will be able to design the prosthetic device and view it three-dimensionally on the CRT screen of the computer. When the design is satisfactory he will instruct the computer to make the prosthetic device, and the computer will issue the appropriate instructions to the stereolithographic components. The skilled man programmes the computer using commands in an appropriate language and then sets the appropriate exposure times and rates for the curable material. To operate the system an image of the prosthetic device is created and a programme is written to drive the stereolithograph to make that device.

The elevator platform can be raised or lowered by appropriate means, e.g. mechanically or hydraulically, and it can have multiple degrees of freedom.

We do not exclude the possibility that the dimensions of the device designed in Step B can be translated into commands for a robot which can amend, eg by sculpting, the shape and/or size of the recipient zone such that the match between the surface thereof and the prosthetic device is improved further.

The present invention is further illustrated by reference to the following Examples which show, by way of example only, certain aspects of the present invention.

### Examples 1 and 2

These Examples illustrate the preparation of solid lamellae in Step E of the preferred process of the present invention.

A photo-curable composition of a methacrylate-ended oligomer prepared as in Example 14 of our EP-A-0 112,650 (hereinafter referred to for convenience as "WB31"), triethyleneglycol dimethacrylate, camphorquinone and N,N-dimethylamino-ethyl methacrylate (in the weight ratio 91:9:0.75:0.75) was sandwiched between two glass plates such that a liquid layer of thickness 1.2 mm was formed. The layer was exposed to an Argon ion laser, at 488 nm, and the hardness of both surfaces of the solid lamella prepared therefrom was tested with a Zwick hardness tester.

The results are shown in Table 1 from which it can be seen that a solid lamella can be formed from a photo-curable composition on exposure to an Argon ion laser.

**TABLE 1**

| Example No | Exposure mJcm⁻² | Vicker Surface Hardness | |
|---|---|---|---|
| | | Top | Bottom |
| 1 | 100 | 0.32 | 0.12 |
| 2 | 350 | 0.4 | 0.19 |
| Top: Surface on which the light is incident | | | |

### Examples 3 and 4

These Examples illustrate the preparation of solid lamellae from an addition-polymerisable composition which comprises a particulate inorganic filler in Step E of the preferred process of the present invention.

The procedure of Examples 1 and 2 was repeated except that the photo-curable composition comprised WB 31, triethyleneglycol dimethacrylate, camphorquinone and N,N-dimethylaminoethyl methacrylate (in the weight ratio 85:15:0.75:0.75 instead of in the weight ratio 91:9:0.75:0.75). A mixture of the photo-curable composition and particles of borosilicate glass (average particle size 20 microns), in the weight ratio 1:1 were sandwiched and exposed as described in Examples 1 and 2.

The result are shown in Table 2 from which it can be seen that a solid lamella can be formed from a photo-curable composition.

**TABLE 2**

| Example No | Exposure mJcm⁻² | Vicker Surface Hardness | |
|---|---|---|---|
| | | Top | Bottom |
| 3 | 100 | 1.1 | 0.2 |
| 4 | 350 | 12.5 | 0.9 |
| Top: Surface on which the light is incident | | | |

### Examples 5 and 6

These Examples illustrate the preparation of solid lamella from an addition-polymerisable composition which comprises a higher concentration of particulate inorganic filler.

The procedure of Examples 3 and 4 was repeated except that in the mixture of photo-curable composition and borosilicate glass the ratio of composition: glass was 30:70 instead of 50:50 by weight. An argon ion laser of power 17.2 mWcm⁻² was used.

The results shown in Table 3 show the Vickers Hardness values obtained from the upper and lower surfaces of 1 cm² plaques, 1.5 mm thick after the exposures indicated

**TABLE 3**

| Example No | Exposure Jcm⁻² | Vickers Hardness^{a} | |
|---|---|---|---|
| | | Upper Surface | Lower Surface |
| 5 | 5.0 | 50.9 | 35.6 |
| 6 | 10 | 49.2 | 38.7 |

| | | | |
|---|---|---|---|
| a: Mean of 4 determinations | | | |

For comparison the surface hardness of a particulate filled component with a modulus similar to that of compacted bone (ie about 20 GPa) and described in our EP-A-0 339 816 has a mean surface hardness of 42.7 VH. Thus it can be seen that plaques of thickness 1.5 mm (ie thicker than the plaques which would typically be used in a production process) can be prepared by the present process having mechanical properties similar to compacted bone.

## Claims

1. A process for the preparation of a prosthetic device which process comprises the steps of:
Step A: generating an image of the recipient zone which is to receive the prosthetic device;
Step B: designing the prosthetic device from the image;
Step C: translating the shape and size of the prosthetic device designed in Step B into commands that are appropriate for controlling a source of electromagnetic radiation;
Step D: delivering the commands from Step C to, and to control, the source of electromagnetic radiation such that a beam therefrom defines the shape and size of the prosthetic device; and
Step E: exposing a fluid addition-polymerisable composition to the beam from Step D such that a solid of the shape and size of the prosthetic device is prepared therefrom;
wherein in Step E of the process the addition-polymerisable composition is exposed to suitable electromagnetic radiation such that a solid lamella representing a cross-section of the device is produced; and
wherein successive adjacent lamellae as they are formed are cohesively integrated such that they define the shape and size of the prosthetic device; and
wherein the prosthetic device is a joint, a complex fracture repair, a bone plate, a dental crown or a bone reconstructure.

2. A process as claimed in Claim 1 wherein the joint is a femural head replacement.

3. A process as claimed in Claim 1 or Claim 2 wherein the polymerisable composition comprises a component having at least two addition-polymerisable carbon-carbon double bonds.

4. A process as claimed in any one of the preceding claims wherein the polymerisable composition comprises at least one particulate inorganic solid.

5. A process as claimed in Claim 4 wherein the particulate inorganic solid comprises mica, talc, alumina, glass or hydroxy apatite.

6. A process as claimed in Claim 4 or Claim 5 wherein the refractive indices of the polymerisable composition and the inorganic solid are matched.

7. A process as claimed in any one of Claims 4 to 6 wherein the polymerisable composition comprises 10-80% by volume particulate inorganic solid.

8. A process as claimed in any one of the preceding claims wherein the electromagnetic radiation used in Step D is visible light.

9. A process as claimed in any one of the preceding claims using computer generated graphics in combination with stereolithography.

10. A process as claimed in any one of the preceding claims comprising a further step to produce a bearing surface or a tissue-compatible surface on the prosthetic device.

11. A process as claimed in any one of the preceding claims further characterised in that the prosthetic device comprises a total hip joint replacement.

12. A process according to any one of Claims 4 to 11 wherein at least a portion of the particulate inorganic solid is radio-opaque.

## Patentansprüche

1. Verfahren zur Herstellung einer Prothesenvorrichtung, wobei das Verfahren folgende Schritte umfaßt:
Schritt A: Erzeugen einer Abbildung der Empfangszone, die die Prothesenvorrichtung aufnehmen soll;
Schritt B: Entwerfen der Prothesenvorrichtung aus der Abbildung;
Schritt C: Übersetzen der Form und Größe der in Schritt B entworfenen Prothesenvorrichtung in Befehle, die geeignet sind, eine elektromagnetische Strahlungsquelle zu steuern;
Schritt D: Übermitteln der Befehle aus Schritt C zur elektromagnetischen Strahlungsquelle und deren Steuerung derart, daß ein von ihr ausgehender Strahl die Form und Größe der Prothesenvorrichtung bestimmt; und
Schritt E: Exponieren einer additionspolymerisierbaren Fluidzusammensetzung dem Strahl von Schritt D derart, daß von ihm ein Festkörper mit der Form und Größe der Prothesenvorrichtung hergestellt wird;
wobei in Schritt E des Verfahrens die additionspolymerisierbare Zusammensetzung derart einer geeigneten elektromagnetischen Strahlung ausgesetzt wird, daß eine feste Lamelle erzeugt wird, die einen Querschnitt der Vorrichtung repräsentiert, und
wobei aufeinanderfolgende benachbarte Lamellen, wenn sie gebildet sind, derart zusammenhängend integiert sind, daß sie die Form und Größe der Prothesenvorrichtung bestimmen; und
wobei die Prothesenvorrichtung ein Gelenk, ein komplexes Ersatzglied für eine Fraktur, eine Knochenplatte, eine Zahnkrone oder eine Knochenrekonstruktion ist.

2. Verfahren nach Anspruch 1, wobei das Gelenk ein Femoralkopfersatz ist.

3. Verfahren nach Anspruch 1 oder 2, wobei die polymerisierbare Zusammensetzung eine Komponente enthält, die wenigstens zwei additionspolymerisierbare Kohlenstoff-Kohlenstoffdoppelbindungen aufweist.

4. Verfahren nach einem der vorhergehenden Ansprüche, wobei die polymerisierbare Zusammensetzung wenigstens einen partikelförmigen anorganischen Feststoff enthält.

5. Verfahren nach Anspruch 4, wobei der partikelförmige anorganische Feststoff Glimmer, Talk, Tonerde, Glas oder Hydroxyapatit enthält.

6. Verfahren nach Anspruch 4 oder 5, wobei die Refraktionsindizes der polymerisierbaren Zusammensetzung und des anorganischen Feststoffs aufeinander abgestimmt sind.

7. Verfahren nach einem der Ansprüche 4 bis 6, wobei die polymerisierbare Zusammensetzung 10-80 Vol.-% an partikelförmigem anorganischem Feststoff enthält.

8. Verfahren nach einem der vorhergehenden Ansprüche, wobei die in Schritt D verwendete elektromagnetische Strahlung sichtbares Licht ist.

9. Verfahren nach einem der vorhergehenden Ansprüche, wobei computererzeugte Grafiken in Verbindung mit der Stereolithographie verwendet werden.

10. Verfahren nach einem der vorhergehenden Ansprüche, wobei das Verfahren einen weiteren Schritt aufweist, um eine tragende Oberfläche oder eine gewebeverträgliche Oberfläche auf der Prothesenvorrichtung zu erzeugen.

11. Verfahren nach einem der vorhergehenden Ansprüche, dadurch gekennzeichnet, daß die Prothesenvorrichtung ein gesamtes Hüftgelenkersatzglied umfaßt.

12. Verfahren nach einem der Ansprüche 4 bis 11, wobei wenigstens ein Teil des partikelformigen anorganischen Feststoffs radioopaque ist.

## Revendications

1. Procédé de préparation d'un dispositif prothétique, qui comprend les étapes consistant :
Etape A : à engendrer une image de la zone réceptrice, qui doit recevoir le dispositif prothétique ;
Etape B : à concevoir le dispositif prothétique à partir de l'image ;
Etape C : à traduire la forme et les dimensions du dispositif prothétique conçu dans l'Etape B en commandes qui sont appropriées à la commande d'une source de rayonnement électromagnétique ;
Etape D : à délivrer les commandes de l'Etape C à, et à commander la source de rayonnement électromagnétique de telle sorte qu'un faisceau provenant de cette source définisse la forme et les dimensions du dispositif prothétique ; et
Etape E : à exposer une composition fluide polymérisable par addition au faisceau de l'Etape D de telle sorte qu'un solide ayant la forme et les dimensions du dispositif prothétique soit préparé à partir de cette composition ;
dans lequel dans l'Etape E du procédé, la composition polymérisable par addition est exposée à un rayonnement électromagnétique convenable de telle qu'une lamelle solide représentant une section transversale du dispositif soit produite ; et
dans lequel des lamelles adjacentes successives, telles qu'elles sont formées, sont intégrées de manière cohésive de telle sorte qu'elles définissent la forme et les dimensions du dispositif prothétique ; et
dans lequel le dispositif prothétique est une articulation, une réparation complexe de fracture, une plaque osseuse, une couronne dentaire ou une structure de reconstruction osseuse.

2. Procédé suviant la revendication 1, dans lequel l'articulation est un remplacement de tête de fémur.

3. Procédé suivant la revendication 1 ou la revendication 2, dans lequel la composition polymérisable comprend un constituant possédant au moins deux doubles liaisons carbone-carbone polymérisables par addition.

4. Procédé suivant l'une quelconque des revendications précédentes, dans lequel la composition polymérisable comprend au moins une substance solide inorganique en particules.

5. Procédé suivant la revendication 4, dans lequel la substance solide inorganique en particules consiste en mica, talc, alumine, verre ou hydroxy-apatite.

6. Procédé suivant la revendication 4 ou la revendication 5, dans lequel on fait correspondre les indices de réfraction de la composition polymérisable et de la substance solide inorganique.

7. Procédé suivant l'une quelconque des revendications 4 à 6, dans lequel la composition polymérisable comprend 10 à 80 % en volume de substance solide inorganique en particules.

8. Procédé suivant l'une quelconque des revendications précédentes, dans lequel le rayonnement électromagnétique utilisé dans l'Etape D consiste en lumière visible.

9. Procédé suivant l'une quelconque des revendications précédentes, utilisant des graphiques générés par ordinateur en association avec la stéréolithographie.

10. Procédé suivant l'une quelconque des revendications précédentes, comprenant une étape supplémentaire pour produire une surface porteuse ou une surface compatible avec les tissus sur le dispositif prothétique.

11. Procédé suivant l'une quelconque des revendications précédentes, caractérisé en outre en ce que le dispositif prothétique comprend une prothèse totale de hanche.

12. Procédé suivant l'une quelconque des revendications 4 à 11, dans lequel au moins une partie de la substance solide inorganique en particules est opaque aux rayonnements.
